Europäisches Patentamt

European Patent Office

Office européen des brevets

(19)

(11) **EP 1 008 339 A1**

(12) **DEMANDE DE BREVET EUROPEEN**

(43) Date de publication:
**14.06.2000 Bulletin 2000/24**

(51) Int Cl.[7]: **A61K 7/025**, A61K 7/027,
A61K 7/48

(21) Numéro de dépôt: **99402789.4**

(22) Date de dépôt: **09.11.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **27.11.1998 FR 9814985**

(71) Demandeur: **L'OREAL**
**75008 Paris (FR)**

(72) Inventeurs:
• **Arnaud, Pascal**
**94240 L'Hay les Roses (FR)**
• **Pradier, François**
**92260 Fontenay Aux Roses (FR)**

(74) Mandataire: **Brédeville, Odile Marie**
**L'Oreal,**
**D.P.I.,**
**6, rue Bertrand Sincholle**
**92585 Clichy Cédex (FR)**

(54) **Composition cosmetique contenant une association d'acide acexamique et de polyols**

(57) L'invention se rapporte à une composition notamment anhydre de soin et/ou de maquillage des lèvres ou de la peau contenant comme actif de soin physiologiquement acceptable de l'acide acexamique solubilisé dans une phase grasse à l'aide d'au moins un polyol liquide à température ambiante et présentant une valeur d'IOB (balance inorganique/organique) allant de 1 à 7. Cette composition peut se présenter sous forme de stick notamment de rouge à lèvres ou de baume à lèvres pour protéger les lèvres ou de stick pour traiter et/ou soigner les mains en particulier contre les intempéries et éviter ainsi leur dessèchement et/ou la formation de gerçures et de crevasses.

**Description**

La présente invention se rapporte à une composition de soin et/ou de traitement des lèvres et/ou de la peau des êtres humains, contenant de l'acide de maquillage acexamique [appelé aussi l'acide acétamido-caproïque ou encore l'acide 6-(acétylamino) hexanoïque], compatibilisé dans une phase grasse à l'aide d'un solvant particulier. Cette composition est utilisable dans le domaine cosmétique ou dermatologique. Plus spécialement cette composition se présente sous forme de gel, de crème ou stick anhydre homogène, éventuellement coloré, renfermant cet actif. En particulier, cette composition permet d'éviter le dessèchement et/ou la formation de gerçure ou de crevasse ainsi que le traitement des gerçures et crevasses existantes. Elle assure, en outre, souplesse et douceur aux lèvres et/ou à la peau traitées ainsi qu'une résorption des oedèmes et une suppression des douleurs notamment labiales.

Pour protéger les lèvres contre le dessèchement, on utilise généralement des baumes à lèvres en stick, contenant des huiles traitantes, généralement d'origine végétale telles que les triglycérides (voir à cet effet le document JP-A-04 360 810). Malheureusement, même en quantité élevée, ces huiles ne permettent pas un traitement suffisant des lèvres, surtout lorsque ces dernières sont gonflées ou pourvues de crevasses ou de gerçures.

Pour remédier à cet inconvénient, la demanderesse propose d'utiliser de l'acide acexamique dans une phase grasse telle que celle généralement utilisée dans les domaines cosmétique et dermatologique. Malheureusement, la formulation d'actif comportant des fonctions très polaires, comme par exemple un groupement carboxylique (COOH), ce qui est le cas de l'acide acexamique, dans des milieux lipophiles et en particulier anhydres est limitée quant au choix des milieux et des quantités d'actif incorporables dans ces milieux, en raison du manque de compatibilité entre l'actif et les corps gras de la composition.

En particulier, l'acide acexamique présente une solubilité dans les corps gras même polaires telle que l'huile de ricin faible (inférieure à 1 % massique). Par ailleurs, du fait de son état pulvérulent à température ambiante, il est possible de l'incorporer dans une phase grasse sous une forme dispersée, mais la faible compatibilité avec celle-ci peut être à l'origine de problèmes de stabilité tels que des cristallisations de l'actif pouvant conférer de mauvaises propriétés d'application et/ou un aspect peu esthétique (aspect rugueux, manque de douceur et de glissant à l'application).

Il subsiste donc le besoin de rechercher un moyen d'améliorer la compatibilité de l'acide acexamique avec les milieux lipophiles et en particulier anhydres.

Les inventeurs ont trouvé, de manière inattendue, qu'une catégorie particulière de polyols constituait un ensemble de très bons solvants de l'acide acexamique (solubilité supérieure ou égale à 2 % massique) et que la solution d'acide acexamique obtenue pouvait être incorporée par solubilisation, dispersion ou émulsification dans une phase grasse telle que celle généralement utilisée dans les domaines considérés.

De façon plus précise l'invention a pour objet une composition de soin de traitement ou de maquillage des lèvres et/ou de la peau d'êtres humains contenant de l'acide acexamique, comme actif de soin physiologiquement acceptable, compatibilisé avec une phase grasse à l'aide d'au moins un polyol liquide à température ambiante et présentant une valeur d'IOB allant de 1 à 7.

L'invention a aussi pour objet l'utilisation d'au moins un polyol liquide à température ambiante et présentant une valeur d'IOB allant de 1 à 7 pour compatibiliser l'acide acexamique avec une phase grasse.

Par « compatibilisation », on entend une solubilisation totale ou partielle ou une dispersion homogène au microscope.

Selon l'invention, on entend par "polyol" toute molécule organique comportant dans sa structure chimique au moins deux groupements hydroxy (OH) libres. Ces polyols sont liquides à température ambiante (25°C) et sont caractérisés par une valeur d'IOB (Balance/Inorganique/Organique) défini comme suit :

$$1 \leq IOB \leq 7$$

et de préférence

$$1,5 \leq IOB \leq 5,5$$

Le paramètre IOB est connu de l'homme du métier à partir d'un certain nombre de publications comme l'article de A. FUJITA Pharm. Bull 2, 163-173 (1954) et les documents J0 9/151109, J08/217639 de Shiseido ou J09/175925 de Kose.

A titre d'exemples de polyols qui vérifient les critères précédents, et qui peuvent être utilisés seuls ou en mélange dans la composition de l'invention, on peut citer :

| Nom | Valeur d'IOB |
|---|---|
| - Propylène glycol | 3,333 |
| - Butylène glycol | 2,500 |
| - Isoprène glycol | 2,222 |
| - Pentylène glycol | 2,000 |
| - Hexylène glycol | 1,818 |
| - PEG-4 (*) | 2,656 |
| - PEG-6 | 2,396 |
| - PEG-8 | 2,266 |
| - Glycérol | 5,000 |
| - Panthénol | 3,125 |

(*) De façon générale, on peut citer les polyéthylène glycols (PEG) ayant de 4 à 8 motifs d'éthylène glycol.

A titre d'exemple de polyols qui ne vérifient pas le critère précédent relatif à l'IOB, on peut citer :

| Nom | Valeur d'IOB |
|---|---|
| - PPG-10 Butane diol | 0,588 |
| - polyglycéryl 3 diisostéarate | 0,511 |
| - Huile de ricin | 0,404 |

L'acide acexamique peut être introduit dans la composition de l'invention suivant plusieurs procédés :

- Il peut être solubilisé dans un ou plusieurs polyols selon l'invention, puis le mélange obtenu est ensuite solubilisé ou dispersé ou encore émulsionné dans la phase grasse de la composition ;
- Le ou les polyols selon l'invention peuvent être, dans un premier temps, solubilisés, dispersés, ou encore émulsionnés dans la phase grasse de la composition, puis l'acide acexamique peut être solubilisé dans le mélange polyols/phase grasse.

L'acide acexamique peut être utilisé notamment à raison de 0,05 à 30 % du poids total de la composition, de préférence de 0,1 à 20 % et mieux de 0,1 à 10 %, et encore mieux de 0,1 à 7 %. Il peut se présenter sous forme libre ou sous forme de sel et notamment sous forme de sel de zinc. De préférence, il est sous forme libre.

Le ou les polyols compatibilisant l'acide acexamique avec la phase grasse peuvent être présents en une quantité assurant la compatibilité avec la phase grasse et notamment allant de 0,1 à 95 % du poids total de la composition et mieux en une quantité allant de 1 à 50 %.

Cette composition peut être utilisée telle quelle ou bien être incorporée dans une composition plus complexe. Elle est notamment non collante au toucher, non grasse et douce à l'application, tout en traitant de manière satisfaisante les lèvres et/ou la peau d'êtres humains sur lesquelles elle est appliquée. Elle peut se présenter sous forme de produits cosmétiques ou dermatologiques notamment anhydres. Les produits de soin des lèvres sont préférentiels. Toutefois la composition de l'invention peut être utilisée pour le traitement de certaines zones de la peau comme les mains.

La composition de l'invention peut se présenter sous la forme solide, pâteuse ou liquide plus ou moins fluide. Elle peut être une émulsion ou une dispersion du type huile-dans-eau ou eau-dans-huile, un gel anhydre, solide ou souple, une phase huileuse liquide, une dispersion de vésicules lipidiques dans une phase aqueuse. De préférence, elle se présente sous forme de composition solide de préférence anhydre, et plus spécialement sous forme de stick.

La phase grasse de la composition de l'invention peut contenir un ou plusieurs corps gras liquides à température ambiante et pression atmosphérique, appelés huiles. Ces huiles peuvent être d'origine animale, végétale, minérale ou synthétique. Elles peuvent être hydrocarbonées, fluorées et/ou siliconées.

Comme huiles utilisables dans la composition selon l'invention, on peut citer notamment :

- les huiles hydrocarbonées d'origine animale telles que le perhydrosqualène ;
- les huiles hydrocarbonées végétales telles que les triglycérides liquides d'acides gras de 4 à 10 atomes de carbone et même de 4 à 28 atomes de carbone comme les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;

- les hydrocarbures linéaires ou ramifiés, d'origine minérale ou synthétique tels que les huiles de paraffine et leurs dérivés, la vaseline, les polydécènes, le polyisobutène hydrogéné tel que le parléam ;

- les esters et les éthers de synthèse notamment d'acides gras comme les huiles de formule $R_1COOR_2$ dans laquelle $R_1$ représente le reste d'un acide gras supérieur comportant de 7 à 29 atomes de carbone et $R_2$ représente une chaîne hydrocarbonée contenant de 3 à 30 atomes de carbone comme par exemple l'huile de Purcellin, l'isononanoate d'isononyle, le myristate d'isopropyle, le palmitate d'éthyl2-hexyle, le stéarate d'octyl2-dodécyle, l'érucate d'octyl2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol comme le tétraiso stéarate de pentaérythrityle ;

- des alcools gras ayant de 12 à 26 atomes de carbone comme l'octyldodécanol, le 2-butyloctanol, le 2-hexyldécanol, le 2-undécylpentadécanol, l'alcool oléique ;

- les huiles fluorées partiellement hydrocarbonées et/ou siliconées ;

- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényltriméthylsiloxy diphénylsiloxanes, les diphényl diméthicones, les diphénylméthyldiphényl trisiloxanes, les 2-phényléthyl triméthyl-siloxysilicates,

- et leurs mélanges.

Ces huiles peuvent représenter de 0,2 à 99,85 % du poids total de la composition, de préférence de 1 à 80 %.

La composition de l'invention peut comprendre, en outre, tout ingrédient usuellement utilisé dans le domaine concerné, tel que l'eau, des colorants hydrosolubles ou liposolubles, des antioxydants, des huiles essentielles, des conservateurs, des parfums, des neutralisants, des polymères liposolubles notamment hydrocarbonés tels que les polyalkylènes ou le polylaurate de vinyle, des gélifiants de phase aqueuse, des gélifiants de phase grasse liquide, des cires, des gommes, des tensioactifs comme le phosphate de trioléyle, des actifs cosmétiques ou dermatologiques additionnels comme par exemple des émollients, des hydratants, des vitamines, de la lanoline liquide, des acides gras essentiels, des filtres solaires lipophiles ou hydrophiles, et leurs mélanges. La composition selon l'invention peut contenir également des vésicules lipidiques de type ionique et/ou non ionique. Ces ingrédients, autres que l'eau, peuvent être présents dans la composition de façon usuelle à raison de 0 à 20% du poids total de la composition.

Bien entendu l'homme du métier veillera à choisir les éventuels ingrédients complémentaires et/ou leur quantité de telle manière que les propriétés avantageuses de la composition selon l'invention ne soient pas, ou substantiellement pas, altérées par l'adjonction envisagée.

La composition selon l'invention se présente avantageusement sous forme épaissie anhydre. Aussi, l'invention se rapporte plus spécialement à une composition anhydre de maquillage ou de soin des lèvres, épaissie contenant au moins un agent épaississant choisi parmi les gélifiants de phase grasse, les cires, les charges et leurs mélanges. Les cires peuvent être hydrocarbonées, fluorées et/ou siliconées et être d'origine végétale, minérale, animale et/ou synthétique. En particulier, les cires présentent une température de fusion supérieure à 45 °C.

Comme gélifiant de phase grasse, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium ; la silice ; les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; les galactomannanes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-HANCE-AG ; les gommes notamment siliconées comme les PDMS ayant une viscosité > 500 000 centistokes. Ces gélifiants sont utilisés par exemple à des concentrations de 0,2 à 15 % du poids total de la composition.

Comme cire utilisable dans l'invention, on peut citer des cires hydrocarbonées, siliconées et/ou fluorées, comportant éventuellement des fonctions ester, hydroxyle, ou thiol. A titre d'exemple, on peut citer la lanoline, la cire d'abeilles, la cire de Carnauba ou de Candelilla, la paraffine, les cires de lignite ou microcristalline, la cérésine ou l'ozokérite ; les cires synthétiques comme les cires de polyéthylène, les cires de silicones comme les alkyl ou alkoxy-diméticones ayant de 16 à 45 atomes de carbone, les cires de Fischer-Tropsch et leurs mélanges.

La nature et la quantité des cires sont fonction des propriétés mécaniques et de textures recherchées. A titre indicatif, la composition peut contenir de 0 à 50 % en poids de cires, par rapport au poids total de la composition et mieux de 5 à 30 %. Ces cires sont, en outre, des agents structurants de la composition.

La phase grasse de la composition peut, en outre, contenir des corps gras pâteux, à savoir des corps gras ayant une température de fusion allant de 25°C à 45°C ou une viscosité allant de 0,1 à 40 Pa.s à 40°C mesurée au Contraves tournant à 60 Hz. Comme corps gras pâteux, on peut citer les lanolines et leurs dérivés, les esters du cholestérol, et les silicones comme les PDMS ayant des chaînes pendantes du type alkyle ou alcoxy ayant de 8 à 24 atomes de carbone.

La composition selon l'invention peut se présenter sous la forme d'une composition dermatologique ou de soin des lèvres et/ou de la peau ou sous forme d'une composition de protection solaire des lèvres. Elle se présente alors sous forme non colorée, contenant éventuellement des actifs cosmétiques ou dermatologiques, autres que l'acide acexamique. Elle peut alors être utilisée comme base de soin pour les lèvres ou la peau (baumes à lèvres ou à mains, protégeant les lèvres ou les mains du froid et/ou du soleil et/ou du vent).

La composition de l'invention peut également se présenter sous la forme d'un produit coloré de maquillage des lèvres, comme un rouge à lèvres ou un brillant à lèvres, présentant des propriétés de soin ou de traitement. Aussi, l'invention a encore pour objet une composition anhydre de maquillage des lèvres épaissie contenant de l'acide acexamique, comme actif de soin physiologiquement acceptable, et une phase grasse renfermant cet actif et un agent épaississant ainsi qu'un polyol pour compatibiliser l'acide.

Bien entendu la composition de l'invention doit être cosmétiquement ou dermatologiquement acceptable, à savoir non toxique et susceptible d'être appliquée sur les lèvres ou la peau d'êtres humains.

La composition de l'invention peut comprendre avantageusement une phase particulaire, généralement présente à raison de 0 à 40 % du poids total de la composition, de préférence de 0,5 à 25 %, et qui peut comprendre des pigments et/ou des nacres et/ou des charges habituellement utilisés dans les compositions cosmétiques ou dermatologiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition et font notamment partie des agents structurants susceptibles de conduire à une forme solide.

Les pigments peuvent être présents dans la composition à raison de 0,05 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium (DC Red N°21 ou FDC Yellow N° 6). Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple).

La composition selon l'invention peut être fabriquée par les procédés connus, généralement utilisés dans le domaine cosmétique ou dermatologique. En particulier, elle peut être obtenue par chauffage des différents constituants à la température de fusion des cires les plus élevées, puis coulage du mélange fondu dans un moule (coupelle ou doigt de gant). Elle peut aussi être obtenue par extrusion comme décrit dans la demande EP-A-667 146.

L'invention a encore pour objet un procédé cosmétique de soin ou de traitement des lèvres et/ou de la peau des êtres humains, comprenant l'application sur les lèvres et/ou la peau, notamment desséchées, gercées ou crevassées, de la composition telle que définie ci-dessus.

L'invention a encore pour objet l'utilisation de la composition définie précédemment pour limiter, voire supprimer, le dessèchement et/ou les oedèmes des lèvres et/ou de la peau, les gerçures, les crevasses, et/ou protéger les lèvres et/ou la peau, des intempéries.

L'invention est illustrée plus en détail dans les exemples suivants. Les pourcentages sont donnés en poids.

**\* Exemples 1 à 7**

Les exemples suivants montrent la supériorité des propriétés solubilisantes des polyols selon l'invention vis-à-vis de l'acide acexamique. Les exemples 1 à 5 sont conformes à l'invention et les exemples 6 et 7 sont des exemples comparatifs.

*Mode opératoire :*

L'acide acexamique vendu sous la référence Acide Acexamique N par la société Sanofi Chimie est solubilisé à 90 °C dans le solvant sous agitation magnétique pendant 15 minutes.

Les solutions sont ensuite refroidies et conservées à température ambiante.

Les limites de solubilité correspondent aux taux massiques maximum pour lesquels on n'observe pas de recristallisation de l'acide acexamique à température ambiante.

| Exemple | Solvant | IOB | Limite de solubilité en %massique de l'acide acexamique à température ambiante |
|---------|---------|-----|-------------------------------------------------------------------------------|
| 1 | Glycérol | 5,000 | 2 % |
| 2 | Propylène glycol | 3,333 | 7 % |
| 3 | Butylène glycol | 2,500 | 7 % |
| 4 | PEG-8 | 2,266 | 2 % |
| 5 | Pentylène glycol | 2,000 | 7 % |
| 6 | PPG-10 Butanediol | 0,588 | 1 % |
| 7 | Huile de ricin | 0,404 | Inférieure à 1 % |

**\* Exemples 8 et 9**

Les exemples suivants montrent la possibilité d'augmenter la solubilité de l'acide acexamique dans l'huile de ricin par la présence de butylène glycol solubilisé.

### Exemple 8

- Huile de ricin          99 %
- Acide acexamique       1 %

_____

100 % massique

### Exemple 9

- Huile de ricin          91,08 %
- Butylène glycol          7,92 %
- Acide acexamique       1,00 %

_____

100 % massique

La composition de l'exemple 8 est caractérisée par une recristallisation à température ambiante de l'acide acexamique alors que pour la composition de l'exemple 9, ce dernier reste solubilisé à température ambiante.

La composition de l'exemple 8 a été réalisée suivant le mode opératoire des compositions des exemples précédents.

La composition de l'exemple 9 a été réalisée en solubilisant dans un premier temps à température ambiante le butylène glycol dans l'huile de ricin à l'aide d'une agitation magnétique. Puis l'acide acexamique est solubilisé à 90°C dans le mélange précédent à l'aide d'une agitation magnétique.

**\* Exemple 10 - Base de soin pour les lèvres**

|  |  | % massique |
|---|---|---|
| | Cire de polyéthylène (Performalen 500 New Phase Technologies) | 6,50 % |
| | Cire de Carnauba | 2,40 % |
| | Tétraisostéarate de pentaérythrytyle | 15,00 % |
| | Erucate d'oléyle | 3,50 % |
| | Isononanoate d'isononyle | 16,00 % |
| | Phényl triméthicone (20 cSt DC556 de Dow Corning) | 10,00 % |
| A | Lanoline acétylée | 23,40 % |
| | Polylaurate de vinyle | 8,00 % |
| | Antioxydant | 0,10 % |
| | Phosphate de trioléyle | 1,00 % |
| | Polyméthylsilsesquioxane (Tospearl 145A) | 4,00 % |
| | Parfum | 0,10 % |
| | Glycérol | 8,00 % |
| | Acide acexamique | 2,00 % |
| | | ————— |
| | | 100,00 % |

*Mode opératoire :*

La phase A est préparée de manière conventionnelle, en fondant les cires à 95-100°C avec les pâteux, les huiles et les additifs.

On ajoute ensuite le glycérol qui est émulsionné à 95-100°C à l'aide d'un agitateur du type Moritz avec une vitesse d'agitation de 3 000 tr/min.

On disperse enfin l'acide acexamique dans l'émulsion à 95-100°C.

Après homogénéisation, on peut couler la composition dans des moules adéquats pour obtenir un baume à lèvres en stick.

Le baume obtenu présente de bonnes propriétés cicatrisantes et protectrices des lèvres.

**\* Exemple 11- Rouge à lèvres**

|  |  | % massique |
|---|---|---|
| A | Cire de polyéthylène (Performalen 500) | 6,50 % |
|  | Cire de Carnauba | 2,40 % |
|  | Tétraisostéarate de pentaérythrytyle | 10,00 % |
|  | Erucate d'oleyle | 2,00 % |
|  | Isononanoate d'isononyle | 10,00 % |
|  | Phényl triméthicone (20 cSt) | 7,00 % |
|  | Lanoline acétylée | 24,00 % |
|  | Polylaurate de vinyle | 8,00 % |
|  | Antioxydant | 0,10 % |
|  | Phosphate de trioléyle | 1,00 % |
|  | Polyméthylsilsesquioxane (Tospearl 145A) | 4,00 % |
|  | Parfum | 0,14 % |
| B | Propylène glycol | 16,00 % |
|  | Acide acexamique | 1,20 % |
| C | Oxyde de titane | 0,80 % |
|  | Oxyde de fer noir | 0,06 % |
|  | FDC Yellow N° 6 Laque d'aluminium | 3,30 % |
|  | DC Red N° 7 Laque de calcium | 2,90 % |
|  | DC Red N° 21 Laque d'aluminium | 0,60 % |
|  |  | 100,00 % |

_Mode opératoire_ :

La phase A est préparée de manière conventionnelle, en fondant les cires à 95-100°C avec les pâteux, les huiles et les additifs.
La phase C est broyée dans la phase A à l'aide d'un broyeur tricylindre.
La phase B est préparée séparément en solubilisant à 90°C l'acide acexamique dans le propylène glycol.
La phase B est ensuite ajoutée au reste de la composition et émulsionnée à l'aide d'un agitateur de type Moritz avec une vitesse d'agitation de 3000 tr/min.
On peut enfin couler la composition dans des moules adéquats pour obtenir un rouge à lèvres en stick ayant de bonnes propriétés traitantes .

**Revendications**

1.   Composition de soin, de traitement ou de maquillage des lèvres et/ou de la peau contenant de l'acide acexamique, comme actif de soin physiologiquement acceptable, compatibilisé avec une phase grasse à l'aide d'au moins un polyol liquide à température ambiante et présentant une valeur d'IOB allant de 1 à 7.

2.   Composition selon la revendication 1, caractérisée en ce que l'acide acexamique est utilisé à raison de 0,05 à 30 % et mieux de 0,1 à 10 % du poids total de la composition.

3.   Composition selon la revendication 1 ou 2, caractérisée en ce que l'acide acexamique se présente sous forme libre.

4.   Composition selon l'une des revendications précédentes, caractérisée en ce que le polyol présente une valeur d'IOB allant de 1,5 à 5,5.

**5.** Composition selon l'une des revendications précédentes, caractérisée en ce que le polyol est choisi parmi le propylène glycol, le butylène glycol, l'isoprène glycol, le pentylène glycol, l'hexylène glycol, les polyéthylène glycols ayant de 4 à 8 motifs éthylène glycol, le glycérol, le panthénol, et leurs mélanges.

**6.** Composition selon l'une des revendications précédentes, caractérisée en ce que le polyol est présent en une quantité allant de 0,1 à 95 % du poids total de la composition et mieux en une quantité allant de 1 à 50 %.

**7.** Composition selon l'une des revendications précédentes, caractérisée en ce que la phase grasse contient au moins une huile liquide à température ambiante choisie parmi les huiles hydrocarbonées d'origine animale, végétale ou minérale, les huiles siliconées et/ou fluorées et leurs mélanges.

**8.** Composition selon l'une des revendications précédentes, caractérisée en ce qu'elle contient au moins une huile choisie parmi le perhydrosqualène ; les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique, l'huile de jojoba, de beurre de karité ; les huiles de paraffine et leurs dérivés, la vaseline, les polydécènes, le polyisobutène hydrogéné tel que le parléam ; l'huile de Purcellin, le myristate d'isopropyle, l'isononanoate d'isononyle, le palmitate d'éthyl2-hexyle, le stéarate d'octyl2-dodécyle, l'érucate d'octyl2-dodécyle, l'isostéarate d'isostéaryle ; l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; le dioctanoate de propylène glycol, le diheptanoate de néopentylglycol, le diisononanoate de diéthylèneglycol ; les esters du pentaérythritol ; l'octyldodécanol, le 2-butyloctanol, le 2-hexyldécanol, le 2-undécylpentadécanol, l'alcool oléique ; les polydiméthylsiloxanes (PDMS) volatiles ou non, linéaires ou cycliques, liquides à température ambiante ; les phényl triméthicones, les phényltriméthylsiloxy-diphényl-siloxanes, les diphényl diméthicones, les diphényl méthyldiphényl trisiloxanes, les 2-phényl-éthyl triméthylsiloxysilicates, et leurs mélanges.

**9.** Composition selon l'une des revendications précédentes, caractérisée en ce qu'elle contient, en outre, au moins une charge particulaire.

**10.** Composition selon l'une des revendications précédentes, caractérisée en ce qu'elle se présente sous forme d'une émulsion ou dispersion huile-dans-eau ou eau-dans-huile, d'une composition anhydre.

**11.** Composition selon l'une des revendications précédentes, caractérisée en ce qu'elle contient, en outre, au moins un ingrédient choisi parmi l'eau, les colorants hydrosolubles ou liposolubles, les antioxydants, les huiles essentielles, les conservateurs, les parfums, les gélifiants de phase aqueuse, les neutralisants, les polymères liposolubles, les gélifiants de phase grasse liquide, les cires, les gommes, les tensioactifs, les actifs cosmétiques ou dermatologiques et leurs mélanges.

**12.** Composition selon l'une des revendications précédentes, caractérisée en ce qu'elle constitue un baume à lèvres ou à mains, un rouge à lèvres ou un brillant à lèvres, ayant des propriétés traitantes.

**13.** Composition selon l'une des revendications précédentes, caractérisée en ce qu'elle se présente sous forme de stick.

**14.** Procédé cosmétique de soin ou de traitement des lèvres et/ou de la peau des êtres humains, comprenant l'application sur les lèvres et/ou la peau de la composition cosmétique selon l'une quelconque des revendications précédentes.

**15.** Utilisation de la composition selon l'une des revendications 1 à 13, pour limiter, voire supprimer, le dessèchement et/ou les oedèmes des lèvres et/ou de la peau, les gerçures, les crevasses, et/ou protéger les lèvres et/ou de la peau des intempéries.

**16.** Utilisation d'au moins un polyol liquide à température ambiante et présentant une valeur d'IOB allant de 1 à 7 pour compatibiliser de l'acide acexamique avec une phase grasse.

EP 1 008 339 A1

**Office européen des brevets**

**RAPPORT DE RECHERCHE EUROPEENNE**

Numéro de la demande

EP 99 40 2789

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 214 357 A (CHINOIN) 18 mars 1987 (1987-03-18) * le document en entier * | 1-16 | A61K7/025 A61K7/027 A61K7/48 |
| A | FR 2 730 931 A (L'OREAL) 30 août 1996 (1996-08-30) * le document en entier * | 1-16 | |
| A | STN, Serveur de Bases de Données, Karlsruhe, DE, Fichier Chemical Abstracts, Vol 123, AN=237894 * résumé * XP002117848 | 1-16 | |
| X,P | EP 0 940 137 A (L'OREAL) 8 septembre 1999 (1999-09-08) * page 3, ligne 35; revendications 1,10 * | 1 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| LA HAYE | 11 avril 2000 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

EP 1 008 339 A1

## ANNEXE AU RAPPORT DE RECHERCHE EUROPEENNE
## RELATIF A LA DEMANDE DE BREVET EUROPEEN NO.

EP 99 40 2789

11-04-2000

| Document brevet cité au rapport de recherche | | Date de publication | Membre(s) de la famille de brevet(s) | | Date de publication |
|---|---|---|---|---|---|
| EP 214357 | A | 18-03-1987 | AT | 57692 T | 15-11-1990 |
| | | | DK | 8503352 A | 24-01-1987 |
| | | | US | 4657923 A | 14-04-1987 |
| FR 2730931 | A | 30-08-1996 | AUCUN | | |
| EP 940137 | A | 08-09-1999 | FR | 2773485 A | 16-07-1999 |
| | | | FR | 2773486 A | 16-07-1999 |
| | | | CN | 1237407 A | 08-12-1999 |
| | | | JP | 11255618 A | 21-09-1999 |

EPO FORM P0460